# EUROPEAN PATENT SPECIFICATION

(11) **EP 0 887 333 B1**
(45) Date of publication and mention of the grant of the patent: **14.11.2001**
(21) Application number: 98810048.3
(22) Date of filing: 27.01.1998
(51) Int. Cl.: C07C 43/295, A01N 31/16, A01N 31/08, C07C 39/367, C07C 49/84

(54) **Process for the production of halogeno-o-hydroxydiphenyl compounds**
Verfahren zur Herstellung von halogenierten o-Hydroxydiphenylverbindungen
Procédé pour la préparation de composés o-hydroxydiphényles halogénés

(30) Priority: 25.06.1997 EP 97810408
(43) Date of publication of application: 30.12.1998
(73) Proprietor: Ciba Specialty Chemicals Holding Inc., 4057 Basel (CH)
(72) Inventor: Kulkarni, Surendra Umesh, Goregaon (E), Mumbai 400 063 (IN); Ekkundi, Vadiraj Subbanna, Goregaon (E), Mumbai 400 063 (IN); Nadkarni, Pradeep Jeevaji, Vile Parle (E), Mumbai 400 057 (IN); Mudaliar, Chandrasekhar Dayal, Matunga, Mumbai 400 019 (IN); Nivalkar, Kishore Ramachandra, Kurar village, Malad East Mumbai 400 097 (IN)

(56) References cited:
- EP-A- 0 857 711
- CH-A- 428 758
- D. C. ATKINSON ET AL: "Substituted (2-phenoxyphenyl)acetic acids with antiinflammatory activity" JOURNAL OF MEDICINAL CHEMISTRY, vol. 26, no. 10, October 1983, pages 1353-1360, XP002034909 WASHINGTON US
- "The Merck Index, Twelfth Edition" 1996 , MERCK & CO. , WHITEHOUSE STATIO, NJ XP002064483 * page 1646, compound no. 9790 *
- W. D. WATSON: "Noncatalytic chlorination of diphenyl ether" JOURNAL OF ORGANIC CHEMISTRY, vol. 44, no. 7, 30 March 1979, pages 1155-1158, XP002048908 EASTON US
- G. A. OLAH: "Synthetic methods and reactions; 127. Regioselective para halogenation of phenols, phenol ethers and anilines with halodimethylsulfonium halides" SYNTHESIS, no. 10, October 1986, pages 868-870, XP002048909 STUTTGART DE
- G. W. YEAGER: "An umpoled synthon approach to the synthesis of 2-aryloxyphenols" SYNTHESIS, no. 1, January 1995, pages 28-30, XP002048910 STUTTGART DE

## Description

The present invention relates to the production of halogeno-o-hydroxydiphenyl compounds having the formula: 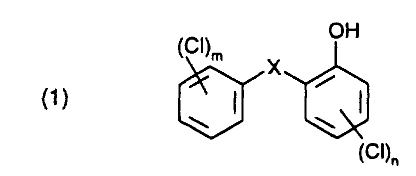 in which
- x: is -O- or -CH₂-;
- m: 1 is 1 to 3; and
- n: is 1 or 2;

The production of halogeno-o-hydroxydiphenyl compounds, especially of 2-hydroxy-2',4,4'-trichlorodiphenylether (Triclosan; compound of formula (3) below), is usually effected by diazotisation and subsequent hydrolysis of 2-amino-2',4,4'-trichlorodiphenylether (TADE; compound of formula (2) below):

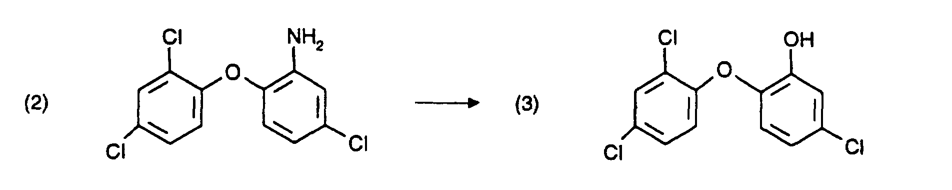

The yields obtained by this production method are unsatisfactory, however, since various chemically competing reactions can occur.

EP-0,857,711-A1 discloses a process for the preparation of halohydroxydiphenyl compounds by acylation of a halogenated benzene compound (first stage), etherification of the acylated compound with a halogenated phenol compound (second stage), oxidation of the etherified compound (third stage) and hydrolysis of the oxidized compound in a fourth stage.

The object of the present invention, therefore, is the provision of an economic process for the production of halogeno-o-hydroxydiphenyl compounds in which undesired side-reactions are suppressed.

According to the present invention, there is provided a four-step process for the production of halogeno-o-hydroxydiphenyl (1) compounds in which, in the first step, a diphenyl compound (4) is chlorinated; in a second step the chlorinated compound (5) is acylated in a Friedel-Crafts reaction and optionally again chlorinated after the acylation; in a third step the acyl compound (6) is oxidized; and in a fourth step the oxidized compound (7) is hydrolyzed; according to the following reaction scheme:

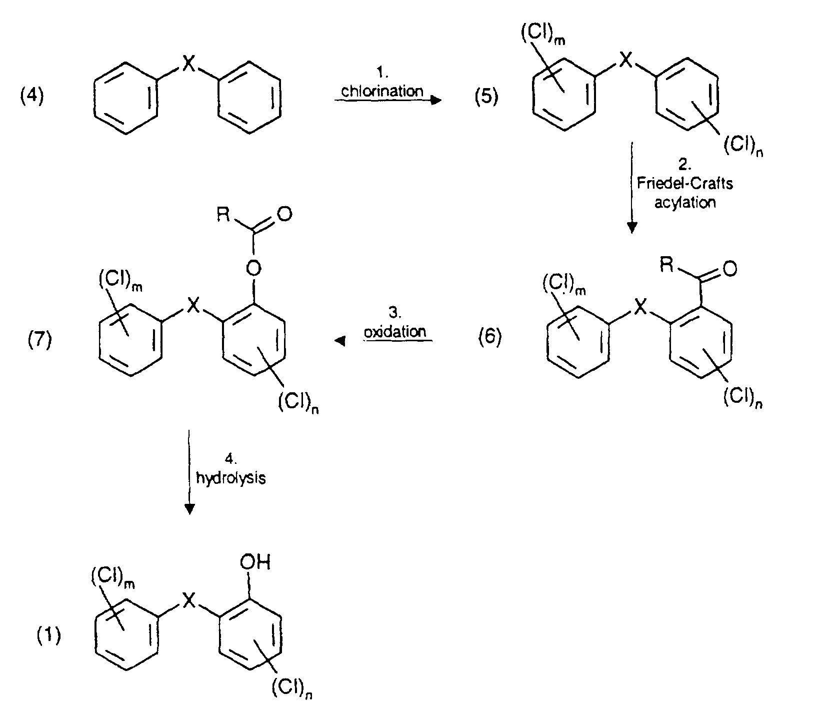

In the above scheme:
- R: is unsubstituted C₁-C₈alkyl or C₁-C₈alkyl substituted by 1 to 3 halogen atoms or hydroxy; or unsubstituted C₆-C₁₂aryl or C₆-C₁₂aryl substituted by 1 to 3 halogen atoms, C₁-C₅alkyl or C₁-C₈alkoxy or combinations thereof;
- X: is -O- or -CH₂-;
- m: is 1 to 3; and
- n: is 1 or 2.

C₁-C₈alkyl denotes branched or unbranched alkyl such as methyl, ethyl, propyl, isopropyl, n-butyl, sec.butyl, isobutyl, t-butyl, 2-ethylbutyl, n-pentyl, isopentyl, 1-methylpentyl, 1,3-dimethylbutyl, n-hexyl, 1-methylhexyl, n-heptyl, isoheptyl, 1,1,3,3-tetramethylbutyl, 1-methylheptyl, 3-methylheptyl, 2-ethylhexyl or n-octyl.

C₁-C₈alkoxy are straight-chain or branched residues such as methoxy, ethoxy, propoxy, butoxy, pentoxy, hexyloxy, heptyloxy or octyloxy.

Halogen denotes fluorine, bromine or, preferably, chlorine.

In the above reaction scheme, in formulae (6) and (7), preferably R is C₁-C₄alkyl, especially methyl.

For the first reaction step, there may be used, as chlorinating agent, e.g. sulfuryl chloride or, preferably, gaseous chlorine. The reaction is preferably conducted in the presence of a catalyst, such as dibenzothiophene, methyl sulfide, propyl sulfide, phenyl sulfide, a Lewis acid, such as aluminium chloride, or mixtures of these compounds. Especially suitable as catalyst for the chlorination reaction according to the invention is a mixture of propyl sulfide and an equimolar amount of aluminium chloride. For the reaction in the first step, the temperature can be selected from within a wide range, e.g. from -10 to 50°C. Preferably, the reaction is conducted at a temperature from 0 to 40°C. The reaction time can also vary within a wide range. Usually the reaction is conducted within a time span of 1 to 48, preferably 2.5 to 10 hours.

The acylation reaction (2. step) is usually conducted in the presence of a Lewis acid, e.g. aluminium chloride. The Lewis acid may be used in amounts of 1 to 3 molar, preferably 1.25 to 2 molar amounts, based on the chlorinated compound of formula (5). A suitable acylating reagent for use in this reaction is an acyl halide, preferably acetyl chloride. Further suitable acylating agents are e.g.

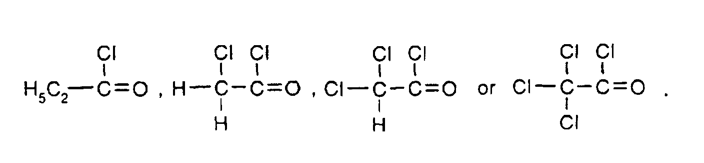

The Lewis acid and acylating reagents are preferably used in equimolar amounts. The reaction may be effected in solvents which are conventionally used for Friedel-crafts reactions, such as halogenated solvents like methylene chloride or ethylene chloride. The reaction time for this reaction step is of secondary importance and can be varied within a wide range, e.g. from 1 to 18 hours.

After the acylation reaction, the reaction mixture may optionally be subjected to a further chlorination reaction, in an analogous manner to the first reaction step, especially if, in the first reaction step, mixtures of differently chlorinated diphenyl compounds are obtained, such as mixtures of 4,4'-dichlorodiphenyl- and 2,4,4'-trichlorodiphenyl compounds. By the subsequent chlorination, uniformly chlorinated acyl compounds are produced.

The chlorination reaction (first step) and the acylation reaction (second step) and the optional further chlorination reaction are preferably conducted in the same reaction vessel, that is in one-pot reactions.

The oxidation of the acyl compound of formula (6) to give the compound of formula (7) (Baeyer-Villiger oxidation), may be effected with various oxidizing agents. Suitable oxidizing agents are, e.g.:
an equimolar mixture of dilute peracetic acid and acetic anhydride in the presence of a catalytic amount of perchloric acid;
an excess of 3-chloro-perbenzoic acid in water;
di-peroxydodecanedioic acid (DPDDA);
a mixture of dilute peracetic acid and acetic anhydride and sulfuric acid;
a mixture of m-chloroperbenzoic acid (MCPBA), trifluoroacetic acid and dichloromethane;
a mixture of sodium borate and trifluoroacetic acid;
a mixture of formic acid, hydrogen peroxide, acetic anhydride, phosphorus pentoxide and acetic acid;
a mixture of acetic acid, hydrogen peroxide, acetic anhydride and phosphorus pentoxide;
a mixture of K₂S₂O₈, sulfuric acid and a 1:1 water/methanol mixture;
a mixture of acetic acid and the potassium salt of monoperoxomaleic acid;
a mixture of trichloromethylene, the potassium salt of monoperoxomaleic acid and sodium hydrogen sulfate;
a mixture of maleic anhydride, acetic anhydride, hydrogen peroxide and trichloromethane;
a mixture of maleic anhydride, a urea-hydrogen peroxide complex and acetic acid; and
Mg-mono-perphthalate.

Preferably there is used for the oxidation, a mixture of maleic anhydride, a urea-hydrogen peroxide complex and acetic acid as solvent.

If desired, a commercially available wetting agent may be added to the oxidizing agent.

The reaction times lie within a wide range and may vary from about 1 hour to about a week, from 4 to 6 days being preferred.

The reaction temperature ranges from -20°C to about 80°C. Preferably, the reaction is conducted at room temperature.

The final hydrolysis to the desired halogeno-o-hydroxydiphenylethers of formula (1) proceeds quantitatively.

Preferably, the process according to the present invention relates to the production of halogeno-o-hydroxydiphenyl compounds having the formula (1) in which
- x: is oxygen, and especially those compounds in which
- m: is 2 and
- n: is 1

Especially preferred is the compound of formula

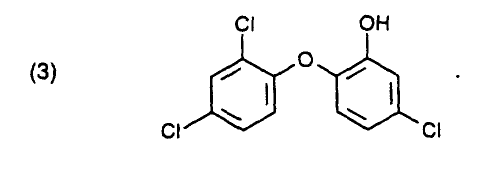

The halogeno-o-hydroxydiphenyl compounds produced according to the invention are insoluble in water but are soluble in dilute sodium- and potassium hydroxide solution and in practically all organic solvents. Due to these solubility properties, the application of the compounds in combatting microorganisms, especially bacteria, and in protecting organic materials against the attack by microorganisms is very versatile. Thus, they can be used, e.g. together with wetting- or dispersing agents, as soaps or synthetic detergent solutions for the disinfection and cleaning of human skin and hands, or they can be applied to these from solid articles in diluted or undiluted form.

The following Examples further illustrate the invention, but without limiting it.

### Example 1a): Chlorination of diphenylether and direct use of the reaction product for reaction with acetyl chloride:

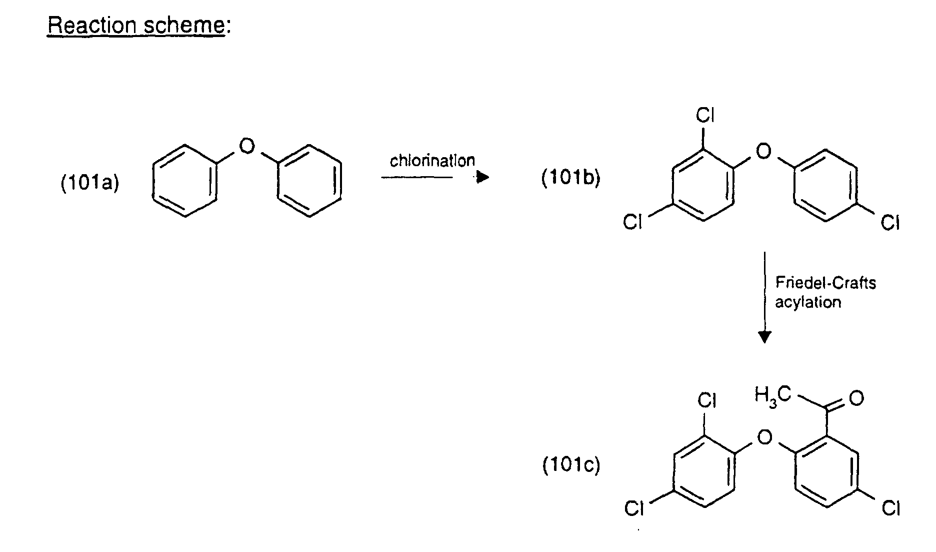

A mixture of 265 g (1.56 mol) of diphenylether (formula 101a), 7.36 g (0.06 mol) of dipropyl sulfide and 7.46 g (0.06 mol) of AlCl₃ are placed in a reaction vessel and melted by stirring and warming to 30°C. The chlorination is conducted by introducing gaseous chlorine at such a rate the reaction mixture can be held at a temperature below 40°C by external cooling. The reaction is monitored using gas- or liquid chromatography. The chlorination is stopped when the content of 2,4,4'-trichlorodiphenylether (compound of the formula 101b) reaches 80 area% (about 6 hours introduction time).

For the acylation, 265 g (3.37 mol) of acetyl chloride are added, dropwise, on to 450 g (3.37 mol) of AlCl₃ in 1100 mls of 1,2-dichloroethane, at 20° to 40°C. The reaction mixture is stirred for 15 minutes at 40°C. Finally, the solution is added, dropwise, to the chlorination reaction mixture in 800 mls of dichloroethane at a temperature of 40°C over about 1 hour. The reaction mixture is then stirred for 10 hours at about 40°C.

The reaction mixture is worked up by treating it with about 4 kg of ice and 550 mls of conc. HCI and extracting it for a short time. An aqueous phase and an organic phase are formed which are separated. After distilling off the solvent from the organic phase, there remains a dark, viscous residue which crystallizes on standing.
Yield: about 490 g of reaction mixture; content of main component about 340 g, corresponding to about 70% of theory, based on diphenylether (formula 101a) used.
Main component: 2-acetyl-4,2,4'-trichlorodiphenylether corresponding to formula (101c).
Composition of the reaction mixture (area % GC or LC): about 70% main component, about 15% 2,2',4,4'-tetrachlorodiphenylether with the rest being unknown compounds.

The reaction mixture can be used directly for the subsequent Baeyer-Villiger oxidation (Example 1b).

### Example 1b): Baeyer-Villiger oxidation

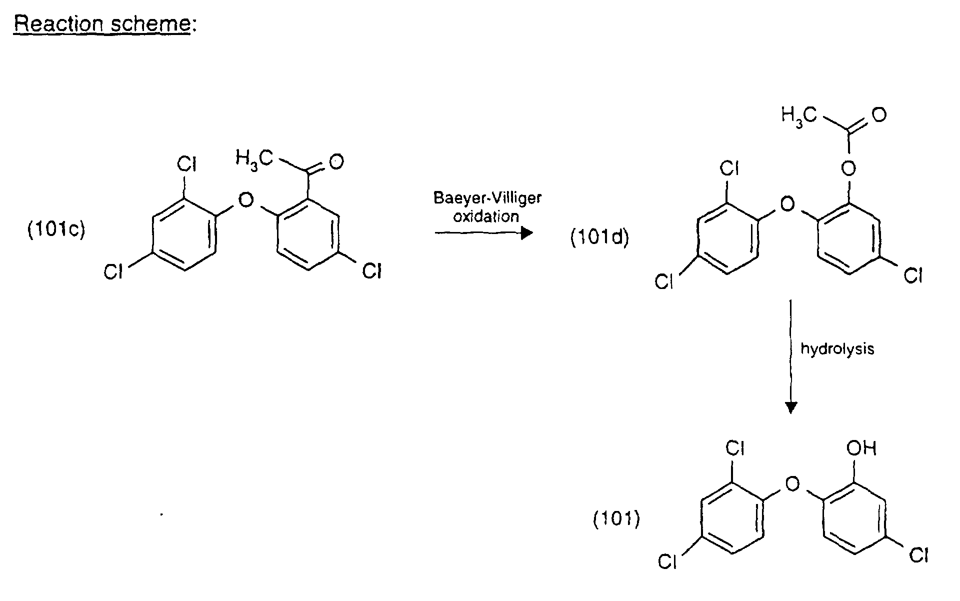

6.32 g of the 2-acetyl-4,2,4'-trichlorodiphenylether corresponding to formula (101c) produced in Example 1a) and 6.88 g of m-chloroperbenzoic acid (MCPBA) are dispersed with a wetting agent in 40 mls of water at 20 to 25°C. The suspension is heated to 80°C and held at this temperature, with vigorous stirring, for 3 hours 30 mls of tetrachloroethylene are added, whereupon two clear phases are formed. The excess peracid is decomposed by adding 0.5 g of sodium hydrogen sulfite, the mixture is adjusted to a pH value of about 8 with NaOH and the aqueous phase (containing m-chloroperbenzoic acid) is separated.

The phenolether of formula (101d) can be recovered, as a white powder of m.pt. 48-49°C, by crystallization from the organic phase

For the hydrolysis, some water is added to the organic phase and the pH value is adjusted to 12 with sodium hydroxide. The end product of formula (101) is obtained from the intermediate product of formula (101d). The pH value is adjusted to about 1 with hydrochloric acid, the aqueous phase is separated and the tetrachloroethylene phase is concentrated.

5.7 g of a yellowish oil are obtained which contains about 80 area % of the compound of formula (101). After recrystallisation from petrol ether, the product is obtained as a white powder having a melting point of 55 to 56°C. The data agree with those of the original compound.

### Example 1c): Alternative Baeyer-Villiger oxidation in anhydrous medium

To a solution of 3 g (10 mmol) of 2-acetyl-4,2,4'-trichlorodiphenylether corresponding to formula (101c) in 20 mls of anhydrous dichloromethane, there are added 4.5 (13 mmol) of m-chloroperbenzoic acid. The mixture is cooled to 0°C and 0.77 ml (10 mmol) of trifluoroacetic acid is added. The reaction mixture is allowed to slowly warm to room temperature. After a reaction time of 8 hours at room temperature, the reaction mixture is cooled with a sodium sulfite solution and washed with a saturated sodium bicarbonate solution. The dichloromethane layer is washed several times with water, dried over anhydrous sodium sulfate and concentrated until an oily residue is obtained. This residue is hydrolyzed by boiling it for 15 hours, under reflux, in 10 mls of 1N NaOH solution. There are obtained 2 g of a crude reaction product which, after acidification, is purified by column chromatography. In this way, there are formed 1.5 g (54% theory) of the compound of formula (101), as a white crystalline powder.

### Alternative hydrolysis

0.9 g of the crude product obtained from the Baeyer-Villiger oxidation is boiled, for 4 hours under reflux, in 5 mls of ethanol which contains a few drops of concentrated HCI. The reaction is monitored by thin layer chromatography. After completion of the reaction, the alcohol is distilled off under vacuum. The oily residue is dissolved in 10 mls of dichloroethane and the solution is repeatedly washed with water. The organic phase is dried over anhydrous Na₂SO₄ and concentrated. There is formed 0.8 g of the crude compound of formula (101). By recrystallisation from petrol ether, there is obtained 0.64 g (70% theory) as a crystalline powder.

### Example 2:

The procedure of Example 1a) is repeated except that the chlorination is conducted in an about 30% solution of diphenylether in 1,2-dichloroethane.

### Examples 3 to 6:

For the acylation reaction described in Example 1a), in addition to acetyl chloride, the acylating agents set out in the following Table 1 can also be used:

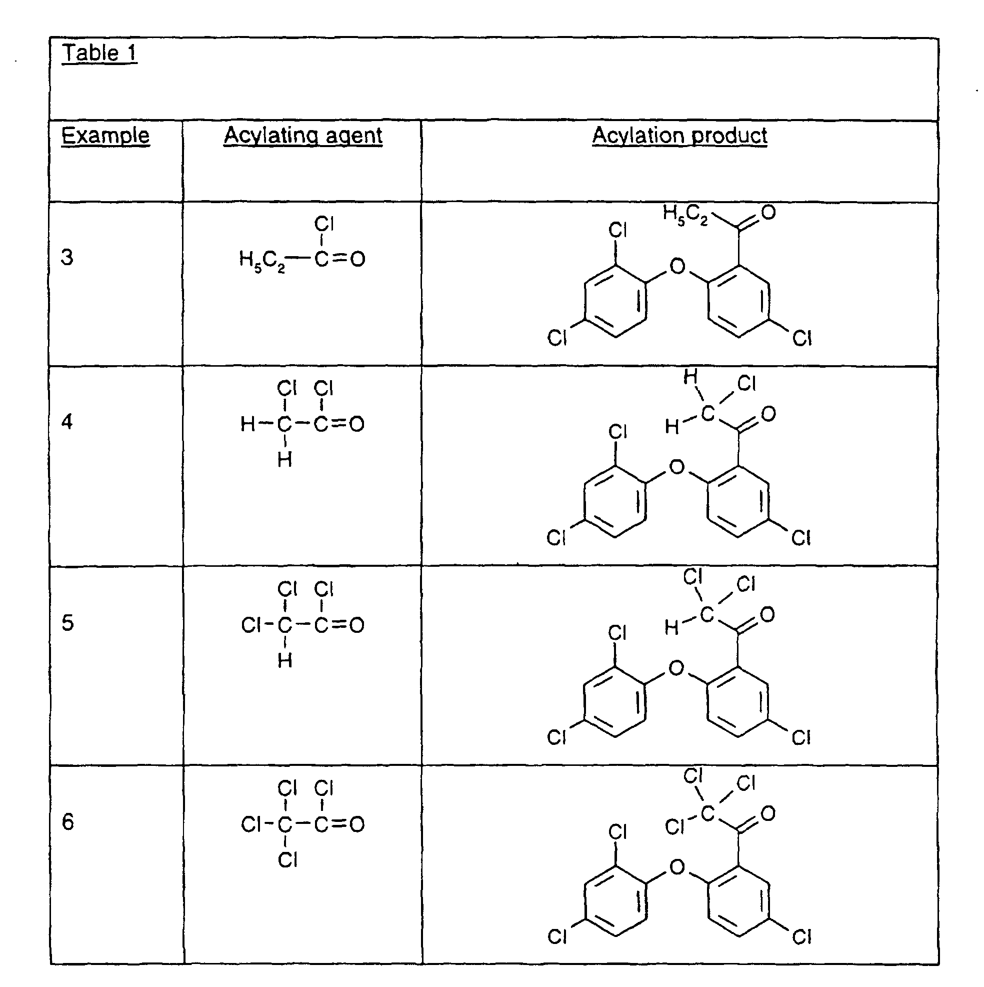

### Example 7: Acylation of a mixture of 2-acetyl-4,2,4'-trichlorodiphenylether and 4,4'-dichlorodiphenylether and a further chlorination reaction

### Example 7a: Acylation

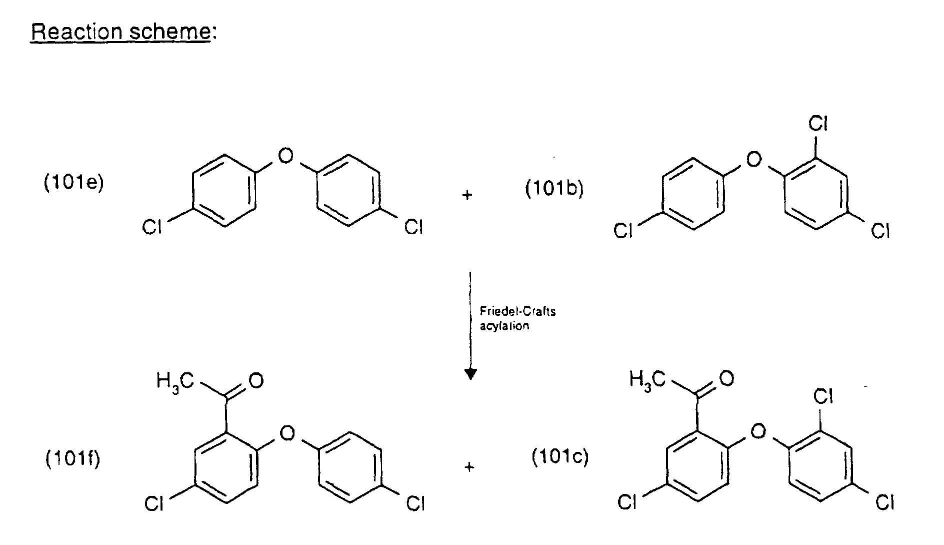

Into a three-necked sulfonation vessel equipped with a pressure-balanced dropping funnel, a nitrogen gas inlet tube, a stirrer and a safety tube, there are placed 480 mls of anhydrous 1,2-dichloroethane and 221.8 g (11.456 mol) of 88% aluminium chloride. The mixture is stirred and cooled in an ice bath under an atmosphere of nitrogen. To this mixture there are added 104 mls of freshly distilled acetyl chloride (114.4 g, 1.456 mol) over a period of 15 to 20 minutes. The exothermic reaction is allowed to cool to room temperature and the mixture is stirred for 30 minutes. A homogeneous dark brown mixture is formed, to which there is added, dropwise, with stirring at room temperature over 15 to 30 minutes, 251.9 g of a mixture containing 2,4,4'-trichlorodiphenylether (79%) (= compound of the formula (101b)) and 4,4'-dichlorodiphenylether (9%) (compound of formula (101b)), dissolved in 480 mls of anhydrous 1,2-dichloroethane. The reaction is monitored by gas chromatography. After stirring for about 15 hours at room temperature, the mixture is added to 500 mls of ice water containing 50 mls of concentrated HCI. After stirring for 15 minutes, the organic phase is separated from the aqueous phase. The aqueous phase is extracted twice with 1,2-dichloroethane, using 100 mls of 1,2-dichloroethane each time. The combined organic phases are washed 6 times with water using 500 mls of water each time and dried over anhydrous sodium sulfate. After removing the solvent, there are obtained 244 g of a mixture containing the compound of formula (101c) and the compound of formula (101f).

This reaction mixture is used for the subsequent chlorination reaction.

### Example 7b: Further chlorination

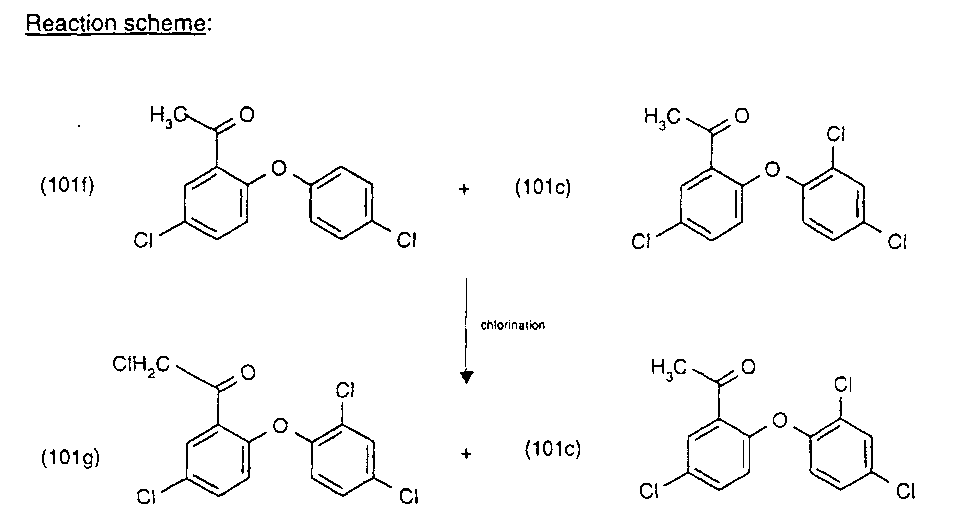

Into a sulfonation vessel equipped with a dropping funnel, a chlorine gas inlet tube, a stirrer, a safety tube and also a purification system for acidic vapors, there are placed 0.077 g (0.65 mmol) of propylsulfide and 88% aluminium chloride in 120 mls of anhydrous 1,2-dichloroethane. Chlorine gas is introduced into this mixture for 15 minutes with stirring. After interrupting the gas supply, there are added, dropwise over 1.5 to 2 hours, 244 g of a mixture conaining 2-acetyl-2,4,4'-trichlorodiphenylether (84.4%) and 2-acetyl-4,4'-dichlorodiphenylether (1.9%), dissolved in 120 mls of anhydrous 1,2-dichloroethane.

Chlorine gas is introduced, with stirring, for one hour. The reaction is monitored using gas chromatography. After completion of the reaction, the mixture is added to 500 mls of ice water containing about 15% HCI. The organic phase is separated and the aqueous phase is washed twice with 1,2-dichloroethane, using 100 mls of 1,2-dichloroethane each time. The combined organic phases are washed five times with a saturated sodium bicarbonate solution, using 200 mls of sodium bicarbonate solution each time, then washed five times with water, using 200 mls of water each time, and dried over sodium sulfate. Finally, the solvent is removed under vacuum. There are obtained 240 g of a crude product containing the compounds of formulae (101c) and (101g)

### Example 8: Acylation

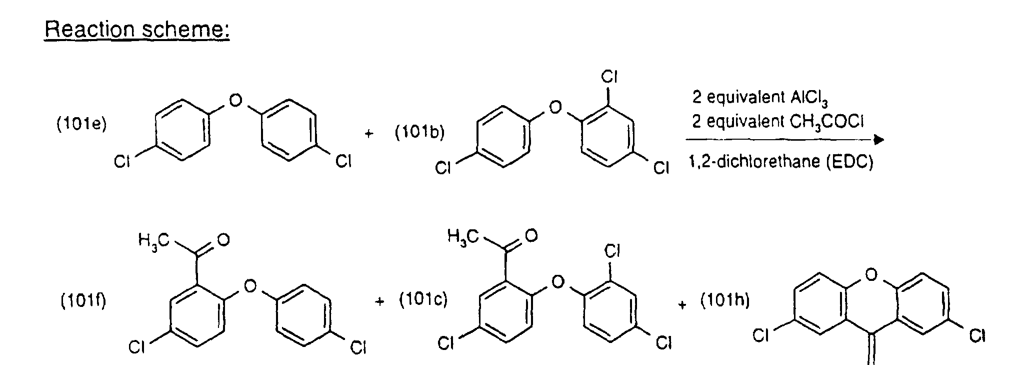

To a three-necked 500 ml round bottom flask equipped with a pressure equalizing dropping funnel, an overhead stirrer and a guard tube, 80 ml of 1,2-dichloroethane (EDC) and 9.85 g of AlCl₃ (0.0664 mol) are added under N₂-atmosphere. The mixture is cooled to 15°C using a water bath, and under stirring 4.7 ml of acetyl chloride (5.18 g, 0.0660 mol) is added dropwise over a period of 30 to 45 minutes. To the above complex 10 g of a mixture of the compound of the formula (101e) (4,4'-dichlorodiphenylether, DCDPE), the compound of the formula (101b) (2,4,4'-trichlorodiphenylether, TCDPE) and 2,2',4,4'-tetrachlorodiphenylether (0.0333 mol w.r.t DCDPE and TCDPE together, TetCDPE) dissolved in 20 ml of EDC Is added dropwise under stirring over a period of 20 minutes at room temperature. On addition of the chlorinating mixture no significant temperature increase is noted. The reaction starts refluxing and the reaction is monitored by GC (using FID and area normalization) at regular intervals. The reaction takes an hour for complete conversion of TCDPE to the compound of formula (101c). During the conversion, 2.3% of the compound of formula (101h) (= xanthene) is also formed.

The table below displays the course of reaction:

**Table 2a:**

| Conversion degree | | | | | |
|---|---|---|---|---|---|
| Time [min] | compound of formula | | | | |
| | (101b) | (101e) + (101b) | (101f) | (101c) | (101h) |
| 30 | 3.0 | 9.6 | 7.8 | 73.5 | 2.0 |
| 60 | 1.0 | 8.9 | 7.2 | 75.0 | 2.3 |

In yet another reaction the amount of EDC is reduced form 10 volumes to 2 volumes and the reaction is done under identical conditions as above.

**Table 2b:**

| Conversion degree | | | | | |
|---|---|---|---|---|---|
| Time [min] | compound of formula | | | | |
| | (101b) | (101e) + (101b) | (101f) | (101c) | (101h) |
| 30 | ≈ 0 | 9.3 | 4.5 | 75.1 | 3.5 |

### Example 9: Acylation

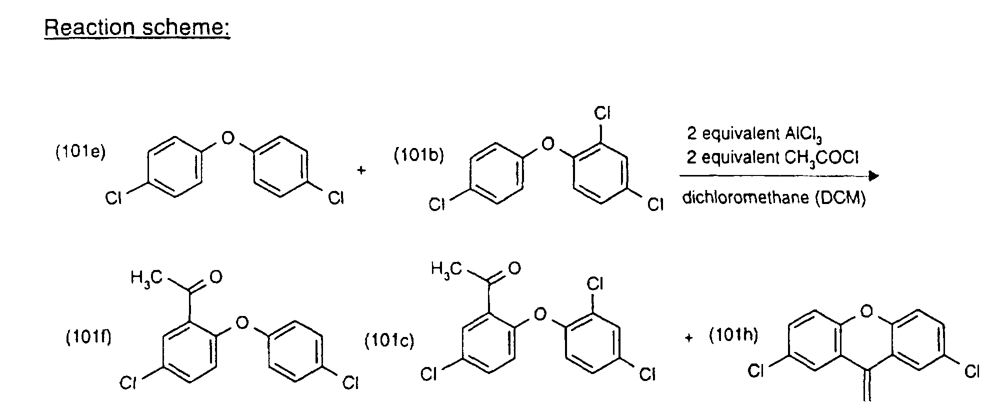

To a three-necked 20 I round bottom flask equipped with a pressure equalizing dropping funnel, an overhead stirrer and a guard tube, 7 1 of dichloromethane (DCM) and 1088.2 g of AlCl₃ (7.328 mol) are added under N2-atmosphere. The mixture is cooled to 15°C using a water bath, and under stirring 522.9 ml of acetyl chloride (575.2 g, 7.328 mol) is added over a period of 20 minutes. During this time the internal temperature of the reaction is raised to about 20°C. The solution is stirred for 10 minutes more when the solution becomes clear. To the above complex 1100 g of a mixture of the compounds of the formulae (101e) (DCDPE), (101b) (TCDPE) and 2,2',4,4'-Tetrachlorodiphenylether (3.664 mol w.r.t DCDPE and TCDPE together) dissolved in 4000 ml of DCM is added under stirring over a period of 20 minutes along with simultaneously heating the reaction mixture. On addition of the chlorinating mixture no significant temperature increase is noted. The reaction will reflux after nearly 1.5 hours. The sampling is done after a regular interval for estimating the extent of reaction on the basis of GC (using FID and area normalization). The reaction takes nearly 22-24 hours for conversion of TCDPE to the compound of formula (101c) (TCADPE). During the conversion, 1.3% of the compound of formula (101 h) (xanthene) is also formed.

The GC data listed in table 3 show the conversion as a function of time:

**Table 3:**

| Conversion degree | | | | | | |
|---|---|---|---|---|---|---|
| Time [h] | compound of formula | | | | | |
| | (101e) | (101b) | (101e) + (101b) | (101f) | (101c) | (101h) |
| 2 | 3.7 | 64.0 | 10.3 | 6.8 | 13.9 | - |
| 6 | 0.5 | 35.4 | 10.2 | 9.6 | 42.9 | 0.3 |
| 11 | ≈ 0 | 10.6 | 9.9 | 10.0 | 67.3 | 0.7 |
| 16 | - | 1.1 | 9.3 | 9.3 | 75.3 | 1.0 |
| 22 | - | 0.2 | 9.0 | 8.7 | 76.6 | 1.3 |

### Example 10: Baeyer-Villiqer oxidation

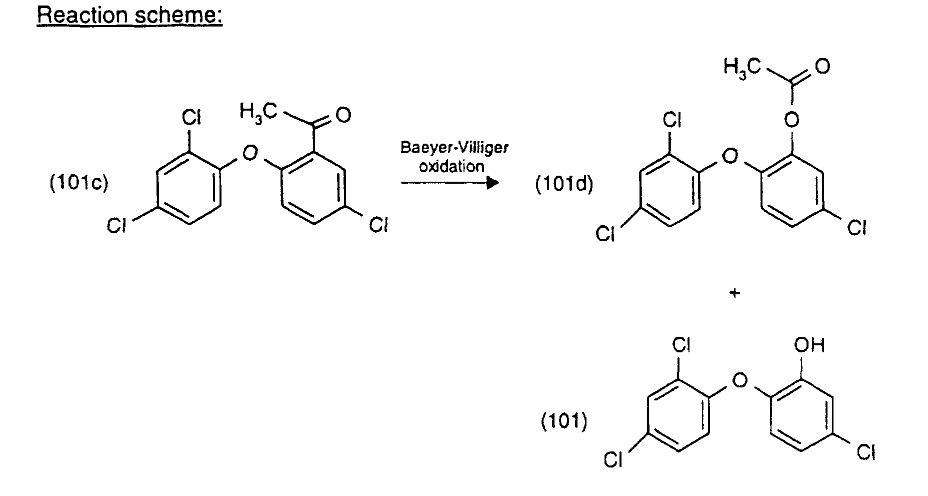

5 ml of acetonitrile is placed in a 50 ml round bottom flask and 0.75 g (0.0079 mol) of ureahydrogenperoxide complex (UHP) and 92 mg (0.0008 mol) of maleic acid are added. To this stirred heterogeneous mixture 0.75 g (0.0076 mol) of maleic anhydride is added in portions at room temperature over 10 minutes. To the above solution 0.25 g (0.0008 mol) of the compound of formula (101c) is added. The reaction is continued further by stirring the reaction at room temperature. The reaction becomes clear after about 45 minutes and is monitored by GC (FID detector and area normalization). After 19 hours the reaction has proceeded to about 40% conversion.

The product distribution is found to be follows (Table 4):

**Table 4:**

| product distribution | | | |
|---|---|---|---|
| | compound of formula | | |
| GC taken after [h] | (101c) | (101d) | (101) |
| 2 | 96.3 | 2.2 | 1.2 |
| 3.5 | 92.0 | 5.6 | 1.4 |
| 19 | 55.7 | 38.8 | 1.8 |

### Example 11: Baever-Villiqer oxidation (the reaction scheme is corresponding to Example 10)

25 ml of trifluoroacetic acid and 5 ml (0.0441 mol) of a 30% hydrogen peroxide solution is placed in a 100 ml round bottom flask. The mixture is stirred for 15 minutes and under stirring at room temperature 5.0 g (0.0158 mol) of the compound of formula (101c) is added. The reaction is continued further by stirring the reaction at room temperature. The solution turns yellowish orange after about 15 minutes and is monitored by GC analysis (FID detector and area normalization).

The product distribution is found to be as follows (Table 5):

**Table 5:**

| product distribution | | | |
|---|---|---|---|
| | compound of formula | | |
| GC taken after [h] | (101c) | (101d) | (101) |
| 1 | 5.2 | 88.2 | 4.4 |
| 2 | 6.2 | 89.3 | 3.5 |
| 3 | 4.6 | 83.7 | 6.9 |
| 5 | 4.1 | 81.4 | 9.1 |
| 18.5 | 19.9 | 61.9 | 9.2 |

### Example 12: Baeyer-Villiger oxidation (the reaction scheme is corresponding to Example 10)

15 ml of acetic acid is submitted in a two-necked 50 ml round bottom flask with a condenser and a dropping funnel. 4 ml (0.0280 mol) of a 70% solution of perchloric acid and 2.0 g (0.0063 mol) of the compound of formula (101c) is added. The homogeneous mixture is heated under stirring to 70-75°C. Using a dropping funnel 4.4 ml (0.0647 mol) of a 50% hydrogen peroxide solution is added dropwise over a period of 30 minutes. After completion the reaction is monitored by GC analysis (FID detector and area normalization).

The product distribution is found to be follows (Table 6):

**Table 6:**

| product distribution | | | |
|---|---|---|---|
| | compound of formula | | |
| GC taken after [h] | (101c) | (101d) | (101) |
| 2 | 42.2 | 6.6 | 46.8 |
| 3.45 | 25.2 | 2.7 | 64.5 |
| 5.5 | 18.9 | 1.6 | 62.0 |
| 21 | 9.0 | 0.3 | 60.9 |

### Example 13: Baeyer-Villiqer oxidation (the Reaction scheme is corresponding to Example 10)

10 ml of water is submitted in a two-necked 100 ml round bottom flask with a dropping funnel. 10 ml of H₂SO₄ is added slowly. 2.0 g (0.0063 mol) of the compound of formula (101c) (2-acetyl-2',4,4'-trichloroacetyldiphenylether) is added and the 50% H₂SO₄ solution is heated to 80°C. The temperature is then further raised to about 130°C. To this solution 3.6 ml (0.318 mol) of 30% H₂O₂ is added dropwise over a period of about 15 to 20 minutes. After completion the reaction is monitored by GC analysis (FID detector and area normalization).

The product distribution is found to be follows (Table 7):

**Table 7:**

| product distribution | | | |
|---|---|---|---|
| | compound of formula | | |
| GC taken after [h] | (101c) | (101d) | (101) |
| 2.5 | 39.2 | 13.6 | 30.3 |
| 19 | 24.6 | - | 54.2 |

## Claims

1. A four step process for the production of halogeno-o-hydroxydiphenyl compounds (1) in which, in the first step, a diphenyl compound (4) is chlorinated; in a second step the chlorinated compound (5) is acylated in a Friedel-Crafts reaction and optionally again chlorinated after the acylation; in a third step the acyl compound (6) is oxidized; and in a fourth step the oxidized compound (7) is hydrolyzed; according to the following reaction scheme: 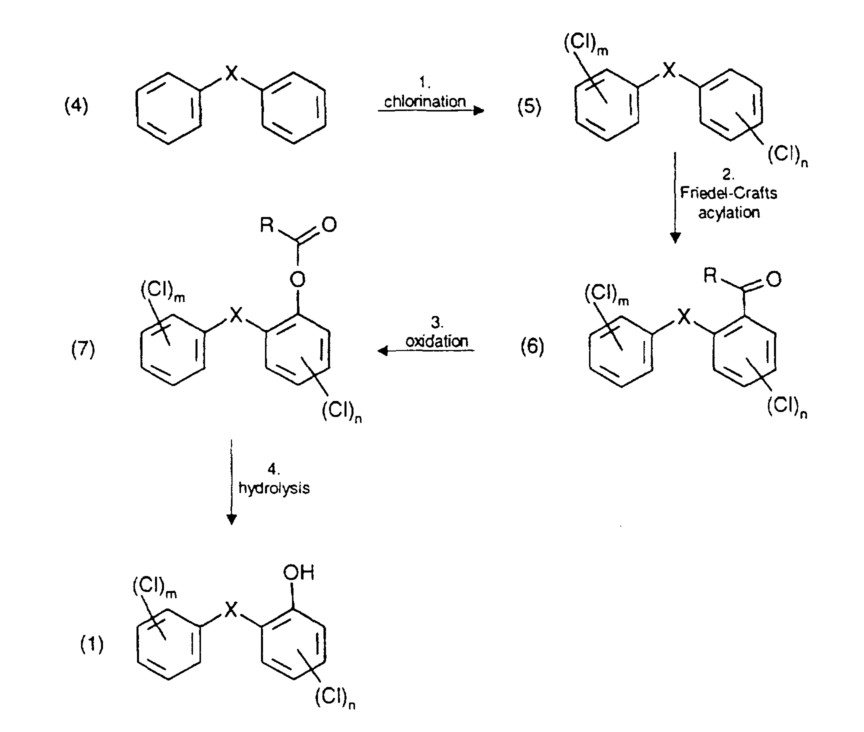 In the above scheme:
R is unsubstituted C₁-C₈alkyl or C₁-C₈alkyl substituted by 1 to 3 halogen atoms or hydroxy; or unsubstituted C₆-C₁₂aryl or C₆-C₁₂aryl substituted by 1 to 3 halogen atoms, C₁-C₅alkyl or C₁-C₈alkoxy or combinations thereof;
X is -O- or -CH₂-;
m is 1 to 3; and
n is 1 or 2.

2. A process according to claim 1 wherein the chlorination (first step) is conducted with elemental chlorine.

3. A process according to claim 1 or 2 wherein the chlorination is conducted in the presence of a mixture of propyl sulfide and an equimolar amount of aluminium chloride.

4. A process according any of claims 1 to 3 wherein a further chlorination follows after the acylation step.

5. A process according to any of claims 1 to 4 wherein the acylation reaction (second step) is conducted in the presence of acetyl chloride and aluminium chloride, whereby acetyl chloride and aluminium chloride are used in equimolar amounts.

6. A process according to claim 5 wherein the acylation reaction is conducted in the presence of an halogenated solvent.

7. A process according to any of claims 1 to 6 wherein the chlorination (first step), the acylation reaction (second step) and optionally the further chlorination are conducted as a one-pot reaction.

8. A process according to any of claims 1 to 7 wherein the oxidation (third step) is conducted with a mixture of maleic anhydride, a urea-hydrogen peroxide complex and acetic acid as solvent.

9. A process according to any of claims 1 to 8 wherein in the formulas (6) and (7)
R is C₁-C₄alkyl.

10. A process according to claim 9 wherein R is methyl.

11. A process according to any of claims 1 to 10 wherein in formula (1)
X is oxygen.

12. A process according to any of claims 1 to 11 wherein in formula (1) m is 2 and n is 1.

## Patentansprüche

1. Ein vierstufiges Verfahren zur Herstellung von Halogen-o-hydroxydiphenyl Verbindungen (1), worin in der ersten Stufe eine Diphenylverbindung (4) chloriert wird; in einer zweiten Stufe die chlorierte Verbindung (5) in einer Friedel-Crafts-Reaktion acyliert wird und wahlweise nach der Acylierung erneut chloriert wird; in einer dritten Stufe die Acylverbindung (6) oxidiert wird; und in einer vierten Stufe die oxidierte Verbindung (7) hydrolysiert wird, gemäß dem folgenden Reaktionsschema: 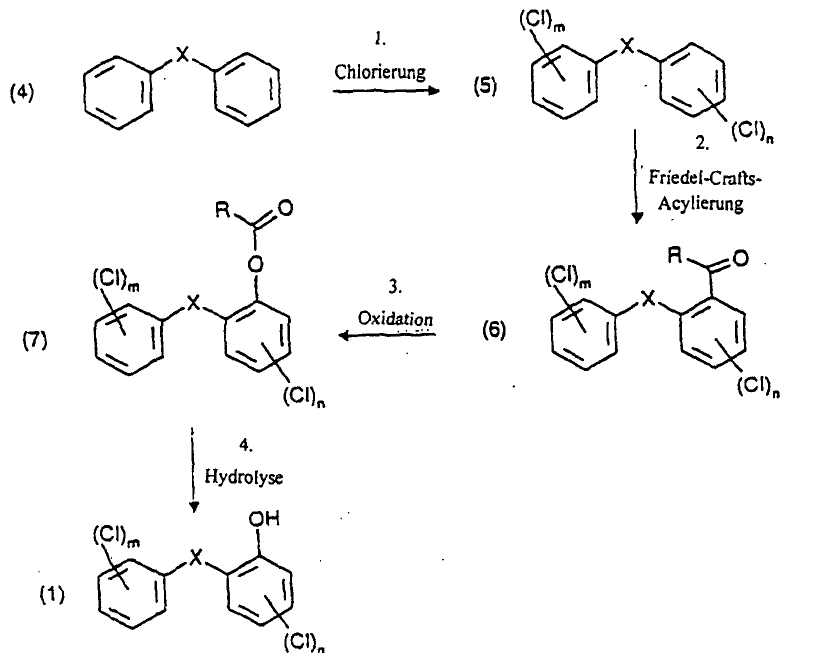 wobei in dem obigen Schema gilt:
R ist; unsubstituiertes C₁-C₈-Alkyl oder C₁-C₈-Alkyl, substituiert mit 1 bis 3 Halogenatomen oder Hydroxy; oder unsubstituiertes C₆-C₁₂-Aryl oder C₆-C₁₂-Aryl, substituiert mit 1 bis 3 Halogenatomen, C₁-C₅-Alkyl oder C₁-C₈-Alkoxy oder Kombinationen davon;
x ist -O- oder -CH₂-;
m ist 1 bis 3; und
n ist 1 oder 2.

2. Verfahren nach Anspruch 1, worin die Chlorierung (erste Stufe) mit elementarem Chlor durchgeführt wird.

3. Verfahren nach Anspruch 1 oder 2, worin die Chlorierung in Gegenwart einer Mischung von Propylsulfid und einer äquimolaren Menge Aluminiumchlorid durchgeführt wird.

4. Verfahren nach mindestens einem der Ansprüche 1 bis 3, worin nach dem Acylierungsschritt eine weitere Chlorierung erfolgt.

5. Verfahren nach mindestens einem der Ansprüche 1 bis 4, worin die Acylierungsreaktion (zweite Stufe) in Gegenwart von Acetylchlorid und Aluminiumchlorid durchgeführt wird, wobei Acetylchlorid und Aluminiumchlorid in äquimolaren Anteilen verwendet werden.

6. Verfahren nach Anspruch 5, worin die Acylierungsreaktion in Gegenwart eines halogenierten Lösungsmittels durchgeführt wird.

7. Verfahren nach mindestens einem der Ansprüche 1 bis 6, worin die Chlorierung (erste Stufe), die Acylierungsreaktion (zweite Stufe) und wahlweise die weitere Chlorierung als eine Eintopfreaktion durchgeführt werden.

8. Verfahren nach mindestens einem der Ansprüche 1 bis 7, worin die Oxidation (dritte Stufe) mit einer Mischung aus Maleinsäureanhydrid, einem Harnstoff-Wasserstoffperoxid-Komplex und Essigsäure als Lösungsmittel durchgeführt wird.

9. Verfahren nach mindestens einem der Ansprüche 1 bis 8, worin in den Formeln (6) und (7) R C₁-C₄-Alkyl ist.

10. Verfahren nach Anspruch 9, worin R Methyl ist.

11. Verfahren nach mindestens einem der Ansprüche 1 bis 10, worin in Formel (1) X Sauerstoff ist.

12. Verfahren nach mindestens einem der Ansprüche 1 bis 11, worin in Formel (1) m 2 ist und n 1 ist.

## Revendications

1. Procédé à quatre étapes pour la production de composés o-hydroxydiphényliques halogénés (1) dans lequel, dans la première étape, un composé diphénylique (4) est chloré ; dans une deuxième étape, le composé chloré (5) est acylé dans une réaction de Friedel-Crafts et facultativement chloré à nouveau après l'acylation ; dans une troisième étape, le composé acylé (6) est oxydé ; et dans une quatrième étape, le composé oxydé (7) est hydrolysé ; selon le schéma réactionnel suivant : 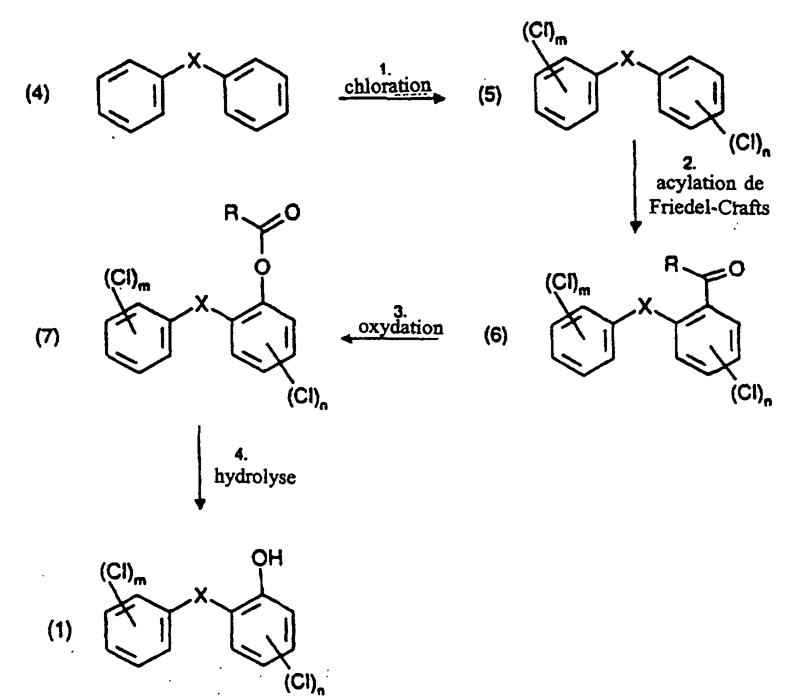 dans le schéma ci-dessus :
R est un groupe alkyle en C₁-C₈ non substitué ou un groupe alkyle en C₁-C₈ substitué par 1 à 3 atomes d'halogène ou groupes hydroxyle ; ou un groupe aryle en C₆-C₁₂ non substitué ou un groupe aryle en C₆-C₁₂ substitué par 1 à 3 atomes d'halogène, groupes alkyle en C₁-C₅ ou groupes alcoxy en C₁-C₈ ou une association d'entre eux ;
x est -O- ou -CH₂- ;
m est 1 à 3 ; et
n est 1 ou 2.

2. Procédé selon la revendication 1, dans lequel la chloration (première étape) est conduite avec du chlore élémentaire.

3. Procédé selon la revendication 1 ou 2, dans lequel la chloration est conduite en présence d'un mélange de sulfure de propyle et d'une quantité équimolaire de chlorure d'aluminium.

4. Procédé selon l'une quelconque des revendications 1 à 3, dans lequel une chloration supplémentaire suit l'étape d'acylation.

5. Procédé selon l'une quelconque des revendications 1 à 4, dans lequel la réaction d'acylation (deuxième étape) est conduite en présence de chlorure d'acétyle et de chlorure d'aluminium, en sorte que le chlorure d'acétyle et le chlorure d'aluminium sont utilisés en quantités équimolaires.

6. Procédé selon la revendication 5, dans lequel la réaction d'acylation est conduite en présence d'un solvant halogéné.

7. Procédé selon l'une quelconque des revendications 1 à 6, dans lequel la chloration (première étape), la réaction d'acylation (deuxième étape) et facultativement la chloration supplémentaire sont conduites comme une réaction dans un seul récipient.

8. Procédé selon l'une quelconque des revendications 1 à 7, dans lequel l'oxydation (troisième étape) est conduite avec un mélange d'anhydride maléique, d'un complexe urée-peroxyde d'hydrogène et d'acide acétique comme solvant.

9. Procédé selon l'une quelconque des revendications 1 à 8, dans lequel, dans les formules (6) et (7), R est un groupe alkyle en C₁-C₄.

10. Procédé selon la revendication 9, dans lequel R est un groupe méthyle.

11. Procédé selon l'une quelconque des revendications 1 à 10, dans lequel, dans la formule (1),
X est l'oxygène.

12. Procédé selon l'une quelconque des revendications 1 à 11, dans lequel, dans la formule (1),
m est 2 et n est 1.
